# EUROPEAN PATENT APPLICATION

(11) **EP 1 417 976 A1**
(43) Date of publication of application: **12.05.2004**
(21) Application number: 02751673.1
(22) Date of filing: 25.07.2002
(51) Int. Cl.: A61K 45/00, A61K 31/56, A61K 31/5377, A61P 27/06, A61P 9/08

(54) **REMEDY FOR GLAUCOMA COMPRISING AS THE ACTIVE INGREDIENT COMPOUND HAVING PI3 KINASE INHIBITORY EFFECT**

(30) Priority: 26.07.2001 JP 2001225606
(71) Applicant: SANTEN PHARMACEUTICAL CO., LTD., Higashiyodogawa-ku, Osaka-shi, Osaka 533-8651 (JP)
(72) Inventor: OTSUKA, Yoshihisa, SANTEN PHARMACEUTICAL CO., LTD., Ikoma-shi, Nara 630-0101 (JP); SHIMAZAKI, Atsushi, SANTEN PHARMACEUTICAL CO., LTD, Ikoma-shi, Nara 630-0101 (JP); MATSUGI, Takeshi, SANTEN PHARMACEUTICAL CO., LTD., Ikoma-shi, Nara 630-0101 (JP); MIYAWAKI, Nobuaki, SANTEN PHARMACEUTICAL CO., LTD., Ikoma-shi, Nara 630-0101 (JP)
(74) Representative: Peaucelle, Chantal
(86) International application number: PCT/JP2002/007528
(87) International publication number: WO 2003/018057

(57) **Abstract**

According to the present invention, a novel pharmacological effect of a compound having a PI3 kinase inhibitory action was found. Study on a novel pharmacological effect of the compound having a PI3 kinase inhibitory action revealed that the compound has an action of reducing intraocular pressure. Therefore, it is found that the compound having a PI3 kinase inhibitory action is useful as a therapeutic agent for glaucoma.

## Description

### Technical Field

The present invention relates to a therapeutic agent for glaucoma comprising a compound having a PI3 kinase inhibitory action as an active ingredient.

### Background Art

Glaucoma is an intractable eye disease which exhibits increased intraocular pressure due to a variety of factors and involves a risk of leading to blindness. Studies have been made for various methods for treating glaucoma. There are three glaucoma-treating methods, i.e., pharmacotherapy, laser therapy and surgery. In the pharmacotherapy, drugs such as β-blockers, prostaglandin analogous drugs, carbonic unhydrase inhibitors, choline agonists, epinephrine analogous drugs and the like have been used.

PI3 kinase (phosphatidylinositol 3-kinase) is an enzyme that phosphorylates a hydroxyl group at the position 3 of an inositol ring that constitutes an inositol phospholipid, and produces phosphatidylinositol 3-phosphate, phosphatidylinositol 3,4-diphosphate and phosphatidylinositol 3,4,5-triphosphate, from a corresponding substrate, i.e., phosphatidylinositol, phosphatidylinositol 4-phosphate and phosphatidylinositol 4,5-diphosphate. PI3 kinase is classified into three groups of types I to III on the basis of the primary structure, regulatory mechanism of the activity, and specificity of the substrate. Extensiveroles have been reported for PI3 kinase, including cell proliferation resulting from platelet-derived growth factor or the like, regulation of cytoskeleton, glucose metabolism by insulin, elongation of neurite, participation to immunocytes, as the dominant roles. Phospholipids with phosphorylated hydroxyl group at the position 3 of the inositol ring by PI3 kinase function as a second messenger which activates serine/threonine kinase such as PDK1 or Akt/PKB in the signal transduction system via receptor stimulation. Furthermore, they also play an important role as a control factor of membrane transport.

JP-A-7-145051 discloses that a PI3 kinase inhibitor is useful in therapy of PI3 kinase-dependent states, particularly neoplasms, and JP-T-9-512553 (the term "JP-T" as used herein means a published Japanese translation of a PCT application) discloses that a PI3 kinase inhibitor is useful in immune response suppression in organ transplantation and autoimmune diseases. However, any application of a PI3 kinase inhibitor to eye diseases has not been referred to in these patent publications.

As described hereinabove, applicability of a compound having a PI3 kinase inhibitory action to an eye disease has not been studied yet, and applied research of a compound having a PI3 kinase inhibitory action on an eye disease raises a greatly interesting subject.

### Disclosure of the Invention

The present inventors elaborately studied the effect of a compound having a PI3 kinase inhibitory action, and consequently found that a compound having a PI3 kinase inhibitory action has an action of reducing intraocular pressure, thereby finding the utility of this compound herein as a therapeutic compound of glaucoma.

### Best Mode for Carrying Out the Invention

The present invention relates to a therapeutic agent for glaucoma comprising a compound having a PI3 kinase inhibitory action as an active ingredient.

Examples of the compound having a PI3 kinase inhibitory action according to the invention include wortmannin (Trends Biochem. Sci., 20, 303-307, 1995), 2-(4-morpholinyl)-8-phenyl-4H-1-benzopyran-4-one (LY294002; JP-T-9-512553), 17β-hydroxy wortmannin and derivatives thereof (JP-A-7-145051), wortmannin 11,17-substituted derivatives (JP-T-10-502373), 4-hexadecyl-3-methoxybutyl phosphonic acid (WO 96/24598), 3-deoxy-D-myo-inositolanalogues (WO 00/00206) and the like.

The action of the intraocular pressure reduction was studied using rabbits and monkeys this time, and it was found that a compound having a PI3 kinase inhibitory action reduces the intraocular pressure. Details are explained in the section of pharmacological tests.

The invention is characterized in that a compound having a PI3 kinase inhibitory action was found to exhibit an effect of the intraocular pressure reduction irrespective of the chemical structure thereof. Thus, the level of the intraocular pressure reduction does not exert an influence on the utility of the invention.

The compound having a PI3 kinase inhibitory action may be administered either orally or parenterally. Examples of the dosage form include tablets, capsules, granules, powders, eyedrops, ophthalmic ointments, injections and the like, and in particular, eyedrops, ophthalmic ointments and injections are preferred. These may be formulated using any widely used technique. Eyedrops may be prepared using an isotonic agent such as sodium chloride or concentrated glycerin, a buffer such as sodium phosphate or sodium acetate, a surfactant such as polyoxyethylene sorbitan monooleate, polyoxyl stearate 40 or polyoxyethylene hydrogenated castor oil, a stabilizer such as sodium citrate or sodium edetate, a presevative such as benzalkonium chloride or paraben, as needed. The pH may be within the range that is acceptable to an ophthalmic formulation. Preferred pH is in the range of 4 to 8. Further, the ophthalmic ointment may be prepared using a vehicle which is widely used such as white soft paraffin, liquid paraffin or the like.

Theinventionalsorelatestoamethodfortreatingglaucoma comprising administering to a patient a therapeutically effective amount of a compound having a PI3 kinase inhibitory action.

The dosage amount may be selected depending on the symptoms, age and the like of patients. The eyedrops may be instilled to patients with a concentration of 0.0001 to 5% (w/v), preferably 0.001 to 3% (w/v), particularly preferably 0.001 to 1% (w/v) once to several times per day.

Formulation Example and results of pharmacological tests are shown below. These Examples are provided for better understanding of the invention, and do not anyhow limit the scope of the invention.

The invention also relates to use of a compound having a PI3 kinase inhibitory action in the manufacture of a therapeutic agent for glaucoma.

### Examples

### [Formulation Example]

General Formulation Example of the eyedrops of the invention is presented below.

| Eyedrops (in 10 mL) | |
|---|---|
| PI3 kinase inhibitor | 1 mg |
| Sodium chloride | 80 mg |
| Polysorbate 80 | 200 mg |
| Sodium dihydrogenphosphate dihydrate | 20 mg |
| 1 N sodium hydroxide | appropriate amount |
| 1 N hydrochloric acid | appropriate amount |
| Sterile purified water | appropriate amount |

### [Pharmacological Test]

In order to examine the influence of the compound having a PI3 kinase blocking action upon the intraocular pressure reduction, measurement of intraocular pressure was conducted using an applanation tonometer or a pneumatic applanation tonometer.

### (Preparation of test compound solution)

LY294002 and wortmannin were provided as test compounds. The former compound was dissolved in methanol to give the concentration of 10⁻¹ M, and thereto was added 2-hydroxypropyl-β-cyclodextrin as a solubilizer. This solution was diluted in physiological saline to give a LY294002 solution having the concentration of 10⁻³ M. The amount of used 2-hydroxypropyl-β-cyclodextrin was determined to make the final concentration thereof to be 4 × 10⁻³ M.

Wortmannin was dissolved in dimethylsulfoxide to give the concentration of 10⁻¹ M for use in the test of administration into anterior chamber, and the obtained solution was diluted in physiological saline to give solutions having the concentration of 10⁻⁴ M and 10⁻⁵ M. Furthermore, dissolution of the compound was conducted in dimethylsulfoxide to give the concentration of 10⁻¹ M for use in the instillation test, and the obtained solution was diluted in physiological saline to give a 0.04284% (10⁻³ M) solution in a suspension state. (Preparation for test of measuring intraocular pressure)

Rabbit intraocular pressure measurement: An electric current source of a tonometer (pneutonometer model 30 classic) was turned on, and calibration was conducted. A 30 G injection needle was attached to a microsyringe, and a test compound solutionoravehiclewasaspiratedtherein. Arabbitpositioner made of aluminum for use in administration into anterior chamber was placed on a table made of aluminum. Further, a rabbit positioner made of wood for use in measurement of intraocular pressure was separately placed on the table made of aluminum.

Monkey intraocular pressure measurement: Measurement of intraocular pressure was conducted using a pneumatic applanation tonometer (Alcon Japan. Ltd.).

### (Administration method and Measurement method of intraocular pressure)

Administration into rabbit anterior chamber: A rabbit was placed on a rabbit positioner made of aluminum for use in administration into anterior chamber, and 0.4% oxybuprocaine hydrochloride was instilled to both eyes with one drop. After fixing an eye lid retractor, the bulbar conjunctiva was held with tweezers, and the needlepoint was passed from corneal limbus into corneal stroma in the aslant direction with the depth of about 2 to 3mm. Thereafter, the needlepoint was pierced into the anterior chamber, where the test compound solution was infused in an amount of 20 µL. After infusing the test compound solution, the needle was slowly drawn out such that the aqueous humor does not leak out. Following the completion of the administration operation, the rabbit was brought back to the cage. The rabbit was brought out from the cage on two hours before the administration, just before the administration, and on 2, 4 and 6 hours after the administration, and was placed on the positioner made of wood. Oxybuprocaine hydrochloride at the concentration of 0.4% was instilled to both eyes, and intraocular pressure was measured after fixing the eye lid retractor. The vehicle was administered in a similar manner.

Administration into monkey anterior chamber: The administration was carried out in accordance with the process for administering to the rabbit. The dosage amount was 10 µL, and the measurement time of the intraocular pressure was on one hour before the administration, just before the administration, andon 2, 4 and 6 hours after the administration.

Test of instillation tomonkey: The test compound solution in an amount of 20 µL was instilled to the eye to be administered. The measurement time of the intraocular pressure was on one hour before the administration, just before the administration, and on 2, 4 and 6 hours after the administration.

### (Results)

Using the intraocular pressure just before the administration as a reference, results are shown by difference (mmHg) between the intraocular pressure of the reference and the intraocular pressure at each time point of the measurement. The results that were obtained when LY294002 was administered into rabbit anterior chamber are shown in Table 1. As obvious from Table 1, the intraocular pressure reduction was observed after the administration of LY294002. Maximum breadth of the intraocular pressure reduction (maximum value of the difference between values of the group administered with the vehicle and the group administered with the test compound) was observed on 6 hours after the administration of 10⁻³ M LY294002, with the value of 3.7 mmHg.

The results that were obtained when wortmannin was administered into a rabbit anterior chamber are shown in Table 2. As obvious from Table 2, the intraocular pressure reduction was observed after the injection of wortmannin. Maximum breadth of the intraocular pressure reduction was observed on 6 hours after the administration of wortmannin, with the value of 3.3 mmHg (10⁻⁵ M) and 6.9 mm Hg (10⁻⁴ M).

The results that were obtained when wortmannin was instilled to a monkey and administered into monkey anterior chamber are shown in Tables 3 and 4, respectively. As obvious from Table 3, the intraocular pressure reduction was observed after the instillation of wortmannin, with the maximum breadth of the intraocular pressure reduction of 2.8 mmHg on 4 hours after the administration. On the other hand, in the case of the administration into the anterior chamber, the maximum intraocular pressure reduction was found on 2 hours after the administration with the value of 4.5 mmHg.

**Table 1**

| Alteration of intraocular pressure after administration of LY294002 into rabbit anterior chamber | | | | | |
|---|---|---|---|---|---|
| | Difference of intraocular pressure (mmHg) | | | | |
| Administration time | 2 hours before administration | Just before administration | 2 hours after administration | 4 hours after administration | 6 hours after administration |
| Vehicle | 1.9 | 0.0 | -0.7 | 0.6 | 1.6 |
| 10⁻³ M LY294002 | 1.9 | 0.0 | -3.2 | -2.7 | -2.1 |

Values in the table represent the mean value of 5 examples and 6 examples of the vehicle group and the 10⁻³ M LY294002 group, respectively.

**Table 2**

| Alteration of intraocular pressure after administration of wortmannin into rabbit anterior chamber | | | | | |
|---|---|---|---|---|---|
| | Difference of intraocular pressure (mmHg) | | | | |
| Administration time | 2 hours before administration | Just before administration | 2 hours after administration | 4 hours after administration | 6 hours after administration |
| Vehicle | 2.2 | 0.0 | 0.9 | 1.5 | 3.9 |
| 10⁻⁵ M wortmannin | 1.2 | 0.0 | -1.5 | -1.0 | 0.6 |
| 10⁻⁴ M wortmannin | 2.2 | 0.0 | -1.2 | -2.8 | -3.0 |

Values in the table represent the mean value of 2 examples, 4 examples and 4 examples of the vehicle group, the 10⁻⁵ M wortmannin group and the 10⁻⁴ M wortmannin group, respectively.

**Table 3**

| Alteration of intraocular pressure after instillation of wortmannin to monkey | | | | | |
|---|---|---|---|---|---|
| | Difference of intraocular pressure (mmHg) | | | | |
| Administration time | 1 hours before administration | Just before administration | 2 hours after administration | 4 hours after administration | 6 hours after administration |
| Vehicle | 1.1 | 0.0 | -0.1 | -0.6 | -0.2 |
| 0.04284% wortmannin | 1.1 | 0.0 | -2.6 | -3.4 | -1.5 |

Values in the table of each group represent the mean value of 5 examples, respectively.

**Table 4**

| Alteration of intraocular pressure after administration of wortmannin into monkey anterior chamber | | | | | |
|---|---|---|---|---|---|
| | Difference of intraocular pressure (mmHg) | | | | |
| Administration time | 1 hours before administration | Just before administration | 2 hours after administration | 4 hours after administration | 6 hours after administration |
| Vehicle | 0.7 | 0.0 | -0.3 | -0.3 | -0.4 |
| 10⁻⁴ M wortmannin | 0.7 | 0.0 | -4.8 | -4.3 | -2.1 |

From the results hereinabove, it was verified that the intraocular pressure is reduced through inhibiting the PI3 kinase.

### Industrial Applicability

Compounds having a PI3 kinase inhibitory action have an excellent action of reducing intraocular pressure, which are useful as a therapeutic agent for glaucoma.

## Claims

1. A therapeutic agent for glaucoma comprising a compound having a PI3 kinase inhibitory action as an active ingredient.

2. The therapeutic agent for glaucoma according to claim 1 wherein the dosage form is an eyedrop.

3. A method for treating glaucoma comprising administering to a patient a therapeutically effective amount of a compound having a PI3 kinase inhibitory action.

4. The method according to claim 3 wherein the administration method is instillation.

5. Use of a compound having a PI3 kinase inhibitory action in the manufacture of a therapeutic agent for glaucoma.

6. Use according to claim 5 wherein the dosage form of said therapeutic agent for glaucoma is an eyedrop.
